# EUROPEAN PATENT SPECIFICATION

(11) **EP 1 894 586 B1**
(45) Date of publication and mention of the grant of the patent: **08.06.2011**
(21) Application number: 07016995.8
(22) Date of filing: 30.08.2007
(51) Int. Cl.: A61M 1/28

(54) **Peritoneal dialysis machine with dual voltage heater circuit and method of operation**
Peritonealdialysemaschine mit spannungsumschaltbarem Heizkreis und Betriebsverfahren dafür
Machine de dialyse péritonéale avec circuit de chauffage à double tension et procédé de fonctionnement

(30) Priority: 31.08.2006 US 513618
(43) Date of publication of application: 05.03.2008
(73) Proprietor: Fresenius Medical Care Holdings, Inc., Waltham, MA 02451-1457 (US)
(72) Inventor: Plahey, Kulwinder S., California (US)
(74) Representative: Martin, Philip John

(56) References cited:
- WO-A-03/099355
- AU-A- 9 184 982
- GB-A- 2 181 311
- US-A1- 2005 080 316

## Description

### TECHNICAL FIELD

This invention relates to peritoneal dialysis systems and related methods,

### BACKGROUND

The present invention relates generally to apparatus for performing peritoneal dialysis on patients with insufficient kidney function, and in particular to heating circuitry for peritoneal dialysis machines designed to accommodate different line or mains voltages, including 110 volts AC (VAC) in the United States and 220. VAC in Europe.

Peritoneal dialysis ("PD") utilizes the patient's own peritoneum (a membranous lining of the abdominal body cavity) acting as a natural semi-permeable membrane. In PD the abdominal or peritoneal cavity of the patient is filled or infused with a sterile aqueous solution called PD solution which is removed or drained after a period of time. PD solution is analogous to dialysate used in hemodialysis; but there are significant differences in the formulations as well as in the process itself. In PD exchanges take place via diffusion and osmosis between the blood stream, i.e., the arterial and venous capillary beds in or near the peritoneum, and the resident reservoir of PD solution itself in the abdomen. Several exchanges may be performed, in a fill-dwell-drain cycle. These exchanges remove toxic waste products, such as urea and creatinine, that each kidney normally excretes into the ureter along with excess water that has built up in the patient's blood stream in the absence of normal kidney function, The kidneys also function to maintain the proper levels of other substances, such as sodium, which are regulated by dialysis to attempt to maintain the proper balance of electrolytes. The diffusion of water and solutes across the peritoneal membrane during dialysis is sometimes called ultrafiltration.

In continuous ambulatory PD (CAPD), a dialysis solution is introduced into the peritoneal cavity utilizing a special permanent catheter inserted through the abdominal wall. After filling, the solution is left in place to accomplish dialysis for a dwell period typically on the order of one or more hours, and then removed by draining it out through the same catheter. The process is repeatable.

Automated PD machines called PD cyclers are designed to control the entire process so that it can be performed at home usually overnight without clinical staff in attendance. This process is termed continuous cycler-assisted PD (CCPD). The cyclers are designed to manage a number of solution bags each typically containing up to 5 liters of PD solution, which the machine pumps or, in so-called gravity systems, allows to flow through a patient line to the patient. But, to avoid thermal shock, the PD solution always has to be heated first to near the patient's body temperature before infusion.

One technique for heating the PD solution is to place a dedicated heater bag on top of a heater tray, equipped with heating coils and a temperature sensor. In this arrangement all fluid going to the patient must come from the heater bag. During the dwell period, the heater bag can be refilled from one of several PD solution bags connected to the machine and warmed so that it will be ready to supply the next fill to the patient.

WO03/099355 describes a method, system and apparatus for performing peritoneal dialysis. The system is electrically insulated so that a grounding connection is not required.

Accommodating varying line voltages encountered world-wide presents a special challenge for the heating circuitry that the present invention is designed to overcome.

### SUMMARY

Briefly, the invention relates to an apparatus for pumping pre-heated fluids between a peritoneal dialysis machine and a patient in order to perform peritoneal dialysis and in particular to an automatic system for detecting 110 or 220 line voltage and safely reconfiguring the connections to the PD solution heater elements in a peritoneal dialysis machine.

According to a first aspect of the invention, there is provided a portable peritoneal dialysis machine as set out in claim 1. The machine comprises a source of PD solution, a patient line for passing PD solution to and from the patient's abdominal cavity, a cycler for delivering a predetermined quantity of PD solution to the patient's abdomen via the patient line, a heater including a series connected heating elements for heating the PD solution before delivering it to the patient a voltage detection circuit connected to detect the line voltage and produce an output indicative of the line voltage to a switch circuit that applies 220 VAC across both elements in series or 110 VAC through the elements in parallel. This arrangement assures that approximately the same wattage is automatically produced by the heater under either 220 VAC or 110 VAC so that the PD solution is heated at approximately the same rate under either voltage.

In one embodiment the heater coils, preferably resistive heating coils, are paired so that under 110 VAC the line voltage is applied to the center tap. The voltage detection circuit preferably has two complementary mutually exclusive logic outputs, one indicating the presence of 220 VAC line current when in one state and the other indicating the presence of 110 VAC line current when in one state.

In one embodiment, the PD solution is heated in a heater bag mounted on a tray on the cycler, the heater being juxtaposed with the tray, for example by embedding resistive heating coils in the tray, to warn the heater bag.

In another embodiment a set of auxiliary heating elements also connected in series and the switch circuit applies 220 VAC across the auxiliary as well as the main heating elements in series or 110 VAC through the auxiliary as well as the main heating elements in parallel. In one embodiment the switch circuit includes a plurality, e.g., four, power switches controlled by a switch control circuit that is responsive to a separately generated control signal that causes the switches to connect the line voltage and neutral to the heating elements to start or stop warming the PD solution. The plurality of switches may include first, second, third and fourth switches, the first switch connecting 220 line voltage when closed to the end of one of the heating elements, the second switch connecting the end of the of the heating elements to neutral when closed, the third switch connecting line voltage to the center tap when closed and the fourth switch connecting the end of the other heating element to neutral when closed, the switch control circuit closing the second, third and fourth switches when the output of the voltage detection circuit indicates that line voltage is 110 VAC, and closing the first and fourth switches when the output of the voltage detection circuit indicates that line voltage is 220 VAC.

Another aspect of the invention includes a method of preparing PD solution for performing peritoneal dialysis as described in claim 11. The method comprises providing a plurality of interconnected heating elements arranged to heat the PD solution prior to infusion, automatically detecting the line voltage and reconfiguring the connection between a plurality of interconnected heating elements with the line voltage and neural in response to the detected line voltage to supply the same wattage for heating the PD solution under at least two substantially different line voltages, and heating the PD solution with the reconfigured heating elements. A patient may be infused with the warmed PD solution. Preferably, the reconfiguring step automatically applies line voltage across the heating elements in series at one line voltage and in parallel at another line voltage.

Advantages of embodiments of the invention include the following. Automatic voltage detection and switching between heater circuits makes it possible to supply and distribute the same PD cycler in all places having voltages in a wide range without modification of the circuitry. One of the advantages of PD cyclers is their portability. Patients do occasionally travel with them. Thus, for example, a dialysis patient is free to travel from the United States to Europe, or vice versa, with his or her regular PD cycler equipped with the heater circuitry of the present invention and not have to worry about the voltage, except possibly for a plug adapter or carrying an alternate power cord.

The details of one or more embodiments of the invention are set forth in the accompanying drawings and the description below. Other features, objects, and advantages of the invention will be apparent from the description and drawings, and from the claims.

### DESCRIPTION OF DRAWINGS

Fig. 1 is a perspective view of a PD cycler on a special cart with a heater bag on the heater tray and additional PD solution bags for more exchanges hanging off the cart.
Fig. 2 is a perspective top view showing the heater tray of the PD cycler of Fig. 1.
Fig. 3 is a rear view of the PD cycler of Fig. 1 showing the ON/OFF switch and power cord.
Fig. 4 is a block diagram with an overview of the AC voltage heater control circuit associated with the heater tray of the PD cycler of Figs. 1 - 3,
Fig. 5 is a block diagram of the heater control circuit of Fig. 4 in the 110 VAC mode.
Fig. 6 is a block diagram of the heater control circuit of Fig. 4 in the 220 VAC mode.
Fig. 7 is a top level block diagram of a specific embodiment of the AC distribution board for implementing the heater circuit system of Figs. 4 - 6.
Fig. 8 is a detailed electrical schematic diagram of the power line interface with voltage detector for the board of Fig. 7. Fig. 8 is divided into three subfigures, Fig. 8A, 8B and 8C, whose interrelationship is indicated by the diagram in Fig. 8 and by the circled letters designating lines that interconnect across two subfigures. The same protocol is used for Figs. 9 and 10.
Fig. 9 is a detailed electrical schematic diagram of the heater control logic for the board of Fig. 7.
Fig. 10 is a detailed electrical schematic diagram of the set of heater relays (triac line switches) for the board of Fig. 7.
Fig. 11 is a representative electrical schematic diagram of one of the triac solid state relays of Fig. 10.
Like reference symbols in the various drawings indicate like elements.

### DETAILED DESCRIPTION

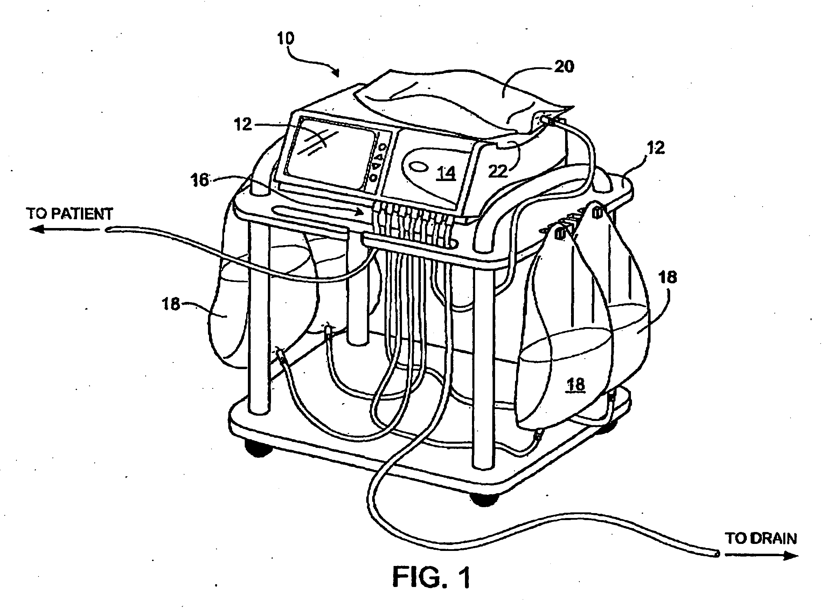

The heater circuit embodiment described below is specifically designed for PD cyclers of the type disclosed in U.S. Patent Application Serial No. 11/069,195, filed February 28, 2005, entitled "Portable Apparatus for Peritoneal Dialysis Therapy," which is incorporated by reference herein in its entirety. The foregoing application is assigned to the same assignee and describes certain details of an embodiment of the PD cycler shown in Fig. 1.

### The Cycler

In Fig. 1, a portable PD cycler 10 is shown seated on top of a cart 12 designed to accommodate the PD solution bags and associated tubing. The front of the cycler 10 includes a control panel 12 that furnishes a user interface designed to be operated by the patient along with a pressurized cassette compartment behind a hinged door 14. The cassette (not shown) includes channels, flexible valve domes and diaphragm covered pumping chambers that are actuated by mating pneumatic valves and pistons interfacing with the cassette compartment to route the flow of PD solution from the bags through the cycler and to the patient and from the patient to a drain. The cassette and cassette compartment are disclosed in more detail in the above-referenced application Serial No. 11/069,195. The cassette itself has tubing connectors 16 arrayed along its bottom edge. The connectors extend beneath the door 14 and are connected to tubing as shown in Fig. 1,

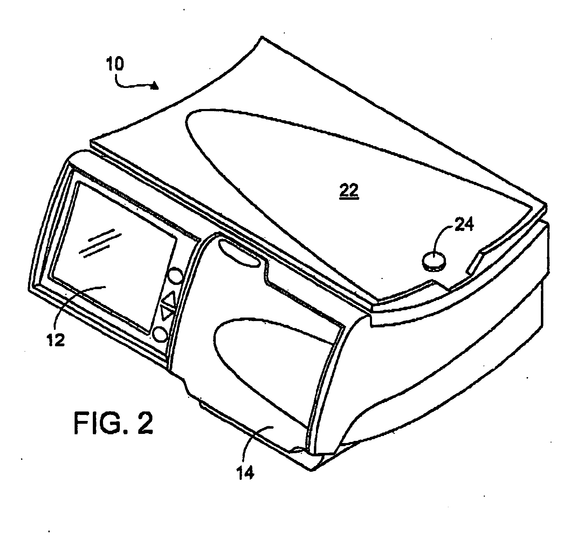

PD solution bags 18 are suspended from fingers on the sides of the cart 12 as shown. A heater bag 20 is shown lying in a shallow concave depression forming the heater tray 22, which is sized and shaped to accommodate a typical 5 L bag of PD solution. The heater tray 22 has a plurality of heating coils (not shown) embedded below the surface. The surface of the tray 22, as better shown in Fig. 2, is slightly inclined downward to the right to assist in emptying the heater bag which is arranged so that the outlet of the heater bag is also at the right side, adjacent to a temperature sensor 24 positioned in the surface of the heater tray 22 to track the temperature of the solution in the heater bag for a thermostatic control circuit that turns the heating coils on and off as needed to maintain the PD solution at the desired temperature. The heater tray 22 is also mounted internally on a support equipped with a load cell (not shown) to provide an electrical signal indicating the weight of the contents of the PD solution bag to tell the cycler control system how full the heater bag is with PD solution.

As shown in Fig, 3, the rear panel 26 of the cycler 10 carries a power cord socket 28 for a detachable power cord 30 with a three prong grounded plug, shown here as an American 110 VAC plug. For use in Europe, a similar power cord with a plug designed for use with 220 VAC line current would ordinarily be simply substituted. Absent the cord one could simply use a suitable 3-prong plug adapter as well. Above the socket 28 is the ON/OFF master power switch 32 for the cycler 10. The rear panel also can include a fan vent 34 and various data ports, for example.

### The Heater Circuit

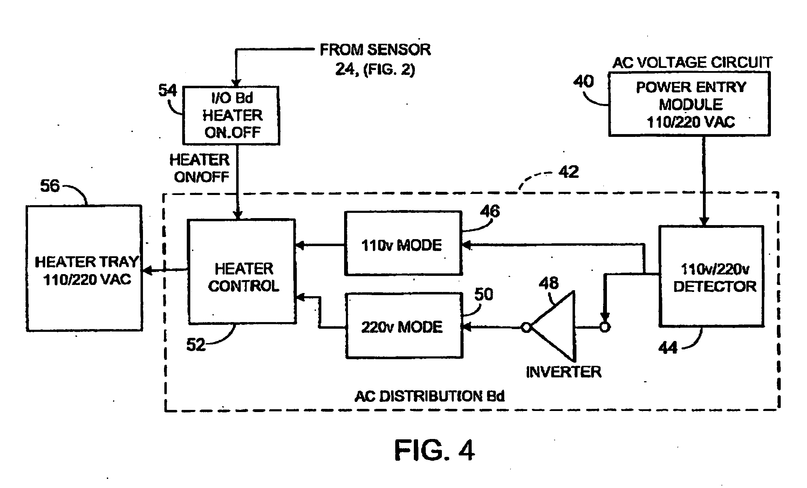

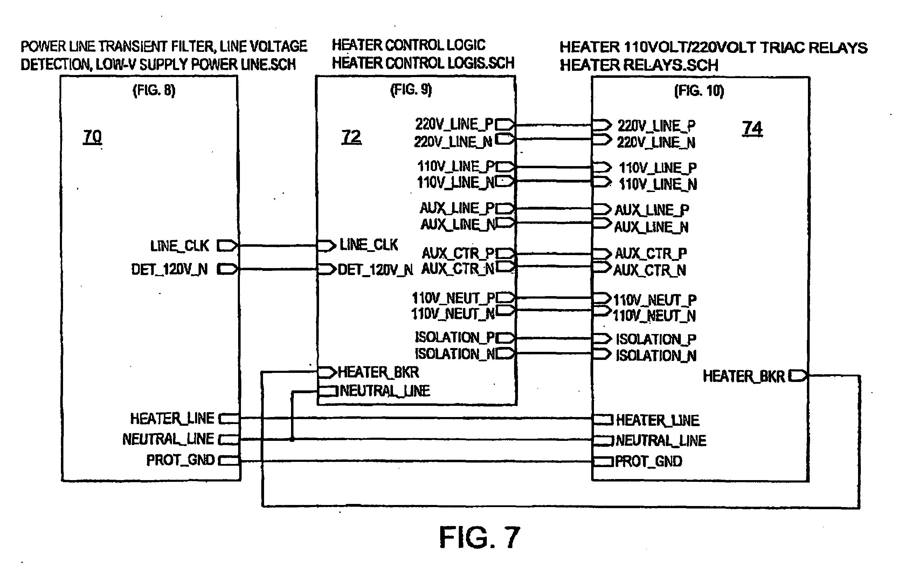

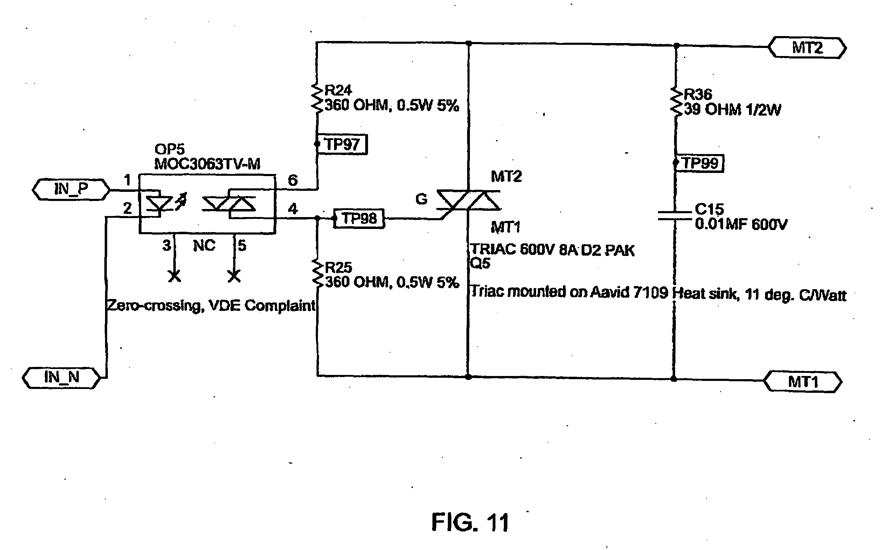

Figs. 4-6 represent an overview of the general operation of the heater circuit under either 110 or 220 VAC, Figs. 7-11 being detailed schematics of an implementation or embodiment of the circuitry for purposes of illustration.

As shown in the upper right portion of Fig. 4, incoming AC line voltage is delivered via a voltage-agnostic power entry module 40 to the AC distribution board 42. Preferably, the circuitry may be designed to handle any voltage between 85 and 265 VAC at 50 to 60 Hz. The AC distribution board includes a 110v/220v detector 44 whose output it a binary logic value for which one level indicates that the line current is 110 VAC and the other level indicates that it is 220 VAC. As shown in Fig. 4 the output of the voltage detector 44 can be passed to the 110v mode controller 46. The output of the voltage detector 44 is also passed via an inverter 48 to a 220v mode controller circuit 50 to insure that only one mode, 110 or 220, can be activated at a time. The output of the active mode controller 46 or 50 energizes a heater control circuit 52 that gates current through the heating coils of the heater tray 56, in different ways depending on the line voltage, in response to ON/OFF signals from the I/O board heater ON/OFF controller 54. Controller 54 is responsive to the temperature sensor 24 to control the ON/OFF cycling of the heater coils to maintain a set temperature in the heater bag while also under the command of the cycler control system to start and stop temperature control in an appropriate energy-efficient manner. For example, once the last bag has been warmed and dispensed to the patient, the heater ON output from the I/O board could be disabled by the cycler control system.

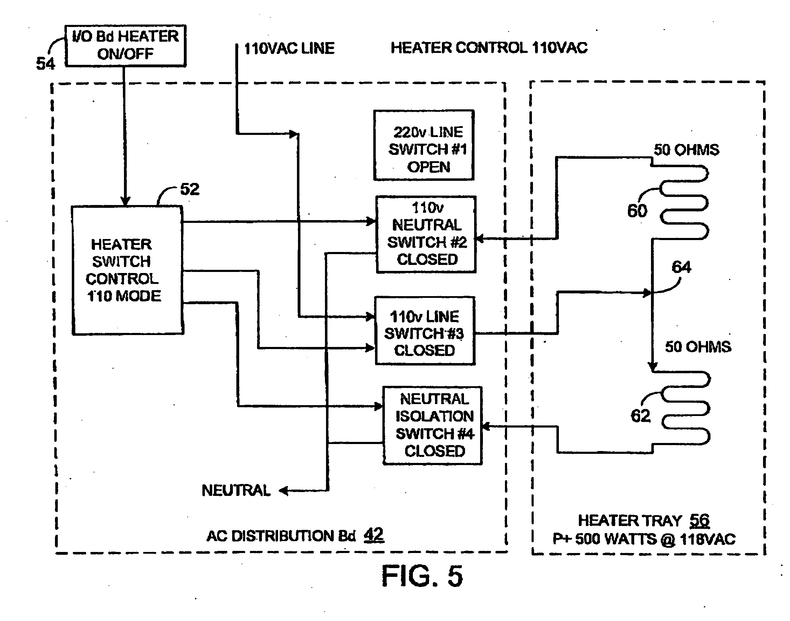

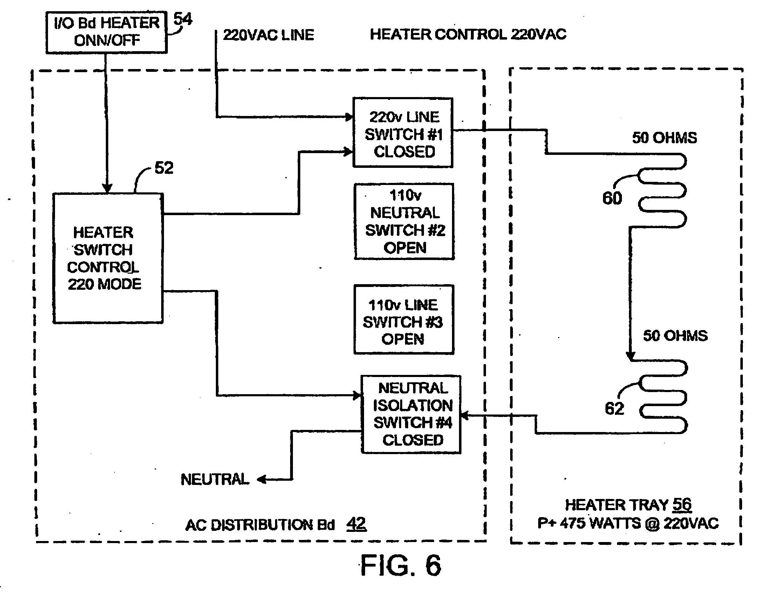

Figs. 5 and 6 show the result of the detection of 110 or 220 line voltage, respectively, on the connections between the power line and the heating coils. Fig. 5 corresponds to the regime when the available line current is 110 VAC. The heater tray 56 includes, by way of illustration, a pair of matched 50 ohm heating coils 60 and 62, connected in series via a center tap 64. Power enters the AC distribution board 42 on two wires or rails, line voltage and neutral. In Fig. 5 the 110 VAC line is shown at the top and the neutral line or rail is shown at the bottom. Line and neutral connections to the heating coils are accomplished via a set of four switches, numbered the same for illustration in Figs. 5 and 6 as switches Nos. 1, 2, 3 and 4 that can either be open (nonconducting) or closed (conducting) depending on signals from the heater switch control 52. To illuminate the different modes, the output lines from the heater switch control 52 in both Figs. 5 and 6 are only indicated for closure of the switches, not for opening. Switch # 1 is the 220v line switch and is open in the 110 mode (Fig. 5). Switch #2 is the 110v neutral switch and is closed in the 110 mode. Switch #3 is the 110v line switch. In 110v mode, corresponding line switch #3 is closed (conducting). The last switch #4 is the neutral isolation switch which is closed in both 110 and 220 modes. Thus, in the 110 VAC mode (Fig. 5) when the I/O board heater controller 54 applies a heater ON signal to the heater control 52 indicating that the cycler control system is commanding the heater to warm up the heater bag and the sensed temperature is below the desired set point, switches Nos. 2, 3 and 4 are closed and switch # 1 is opened. This switch configuration accomplishes the following in the 110 v mode: the 110 VAC line is connected to the center tap 64 via switch #3 while then distal ends of the heater coils 60 and 62 are both connected to neutral via switches Nos. 2 and 4. This configuration passes full 110 VAC current in opposite directions through the respective 50 ohm coils to produce approximately 500 watts of power at 118 VAC. Accordingly when in the 110 v mode, switch #3 closes becoming the center tap/line voltage and switches 2 and 4 close, becoming the neutral return paths, also thereby putting the heater coils 60 and 62 in parallel.

Alternatively, as shown in Fig. 6, in the 220v mode, switches Nos. 1 and 4 are closed by the heater switch control 52. Switch #1 supplies line voltage to the end of coil 60 and switch #4 connects the opposite end of the other coil 62 to neutral return, thus putting line voltage across both heater coils in series, i.e., the 220 VAC current (in one direction) flows through coil 60 then through coil 62 and then returns to neutral (ground). This configuration can generate approximately 475 watts at 220 VAC.

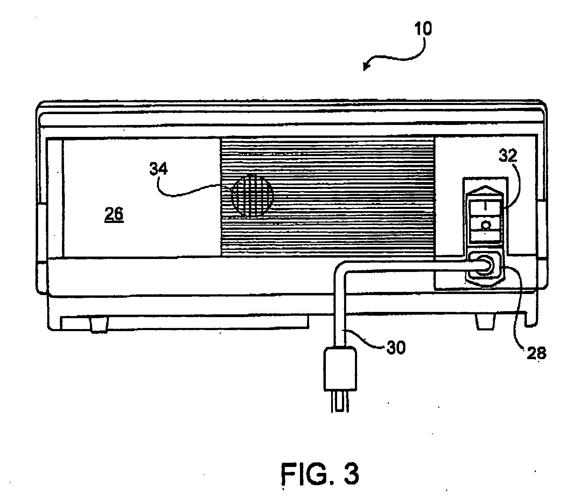

Figs. 7-11 illustrate a preferred specific embodiment of an AC distribution board for the PD heater tray voltage-switched control system developed for a specific heater coil arrangement consisting of two pairs of coils, one designated as an optional auxiliary heater coil pair, for example, of lesser resistance for finer control of the heater bag temperature. Fig. 7 is a top level block diagram of the AC distribution board showing the relationship and signal paths between the three major functional blocks of the circuitry. First, power line circuit 70 filters the AC line, detects the line voltage and provides low voltage DC supply power at 5 and 18 VDC for internal circuit operation. The middle block heater control logic 72 corresponds to heater mode control 52 in Figs. 4- 6, taking its cue from the detected voltage level to provide logical outputs to operate the switches to make the series or parallel connection of the heater coils to line voltage and neutral. The last block, shown on the right in Fig. 7, comprises the heater relays that, under control of the heater control logic block 72, pass current to either the center tap or one end of the series connected coils. But first current must pass through a circuit breaker built into the heater coil assembly (not shown). An additional protective ground is provided which is connected to the longer grounding pin on the standard three-prong plug on power cord 30 (Fig. 3)

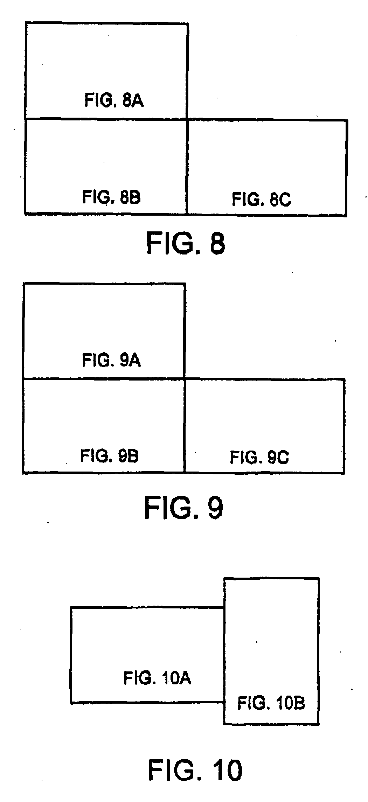

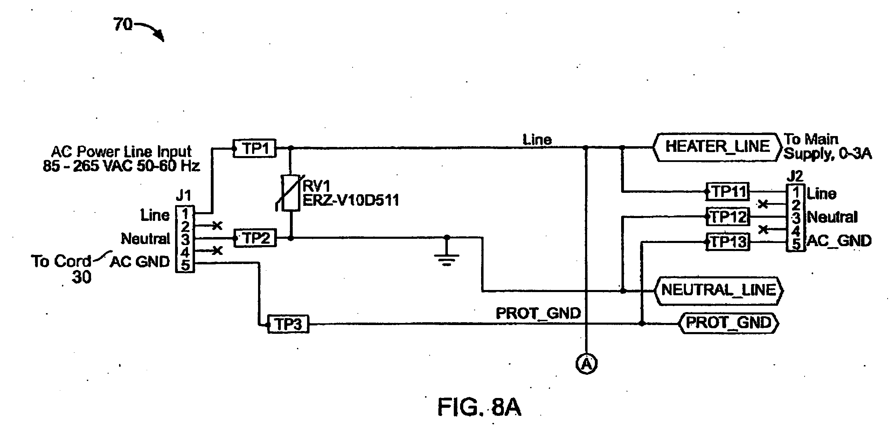

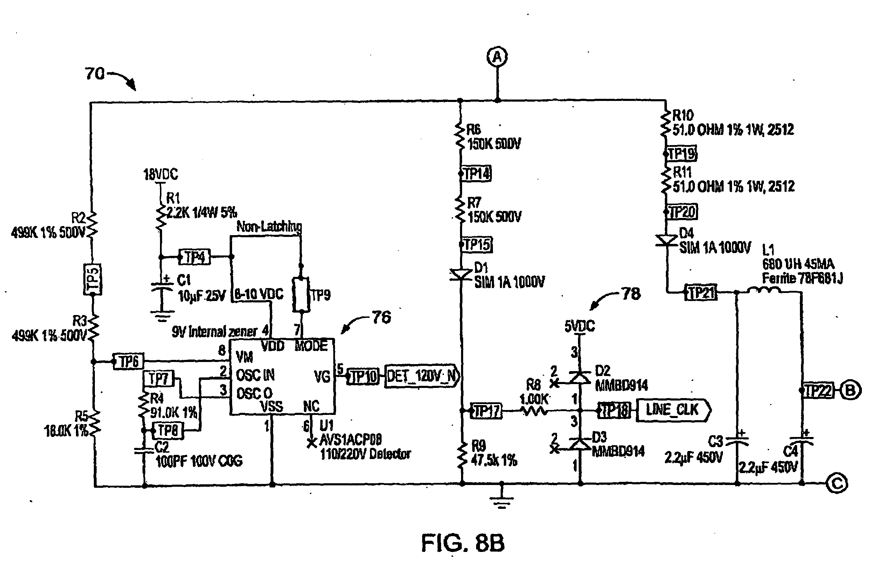

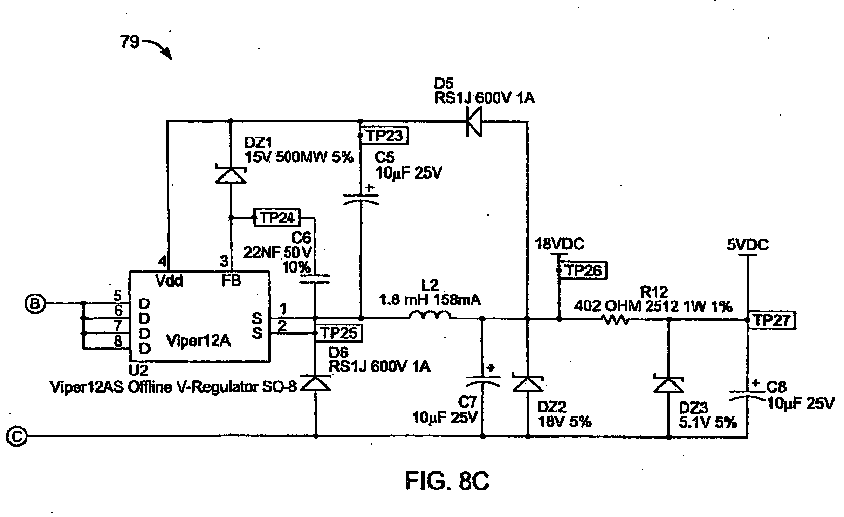

As shown in Fig. 8 the power line interface connects via connector J2 to the power cord 30. LINE and NEUTRAL are connected via protective Zener breakdown diode as shown to furnish the HEATER LINE and NEUTRAL LINE which are fed to both the heater control logic circuit 72 and the relay control circuit 74 by which they are connected directly to the heating coils. LINE is tapped by line A (circled) in Figs. 8A and 8B for the voltage detector 76 and line frequency CLK 78 circuits in Fig. 8B and the 5 and 18 volt DC power supplies 79 in Fig. 8C. The heart of the voltage detector 76 is an integrated circuit U1 that produces the output designated DET_120V_N whose logic value is low when LINE is 110 VAC.

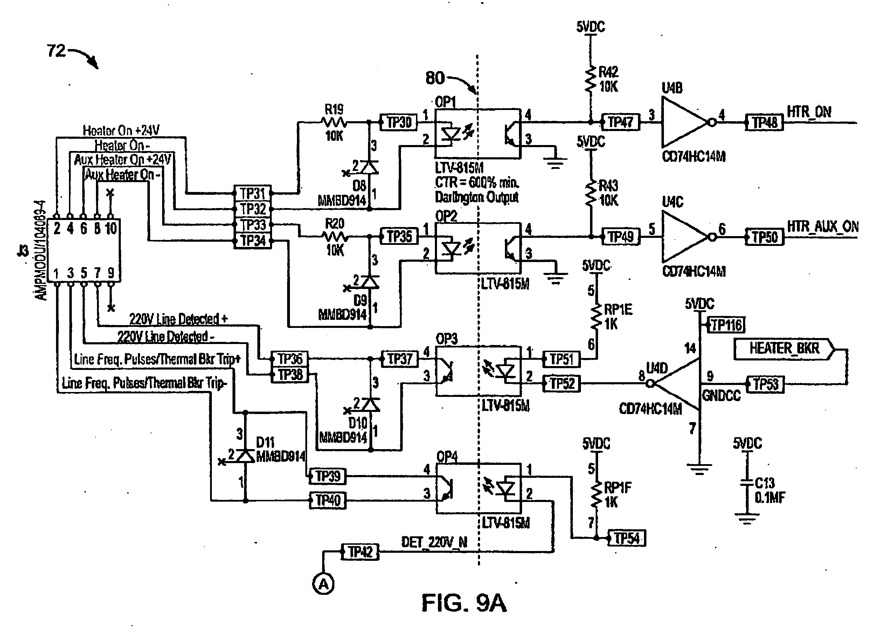

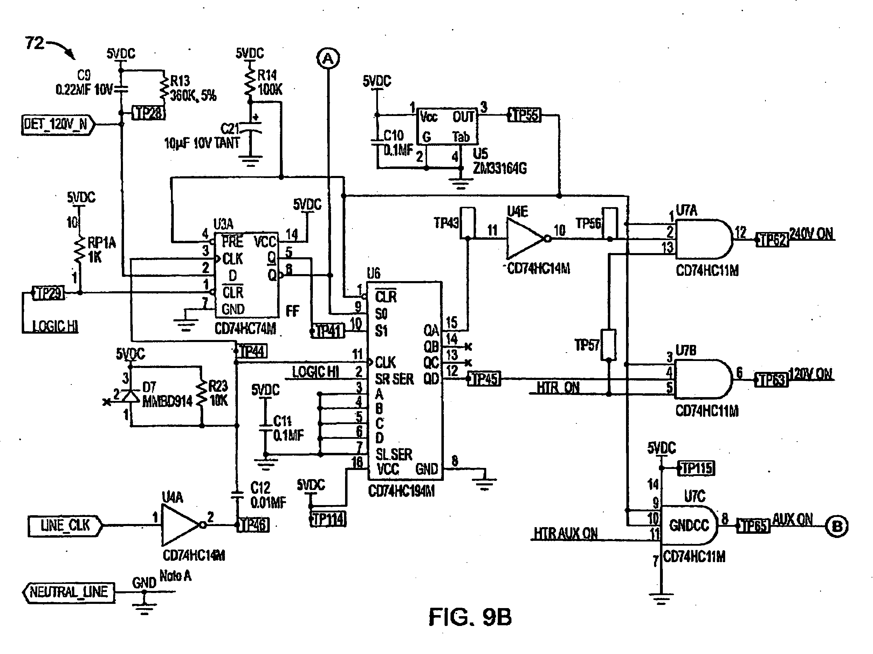

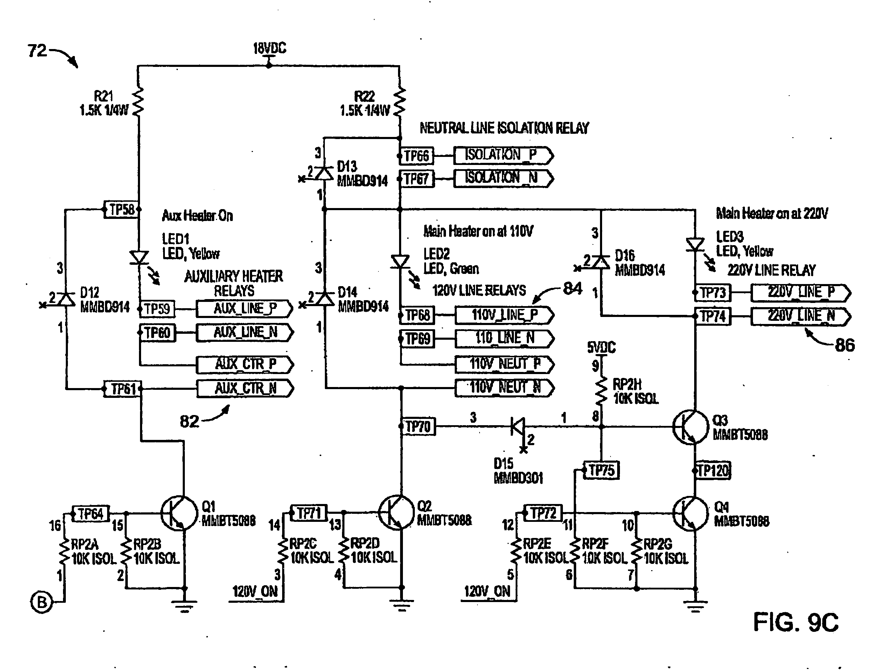

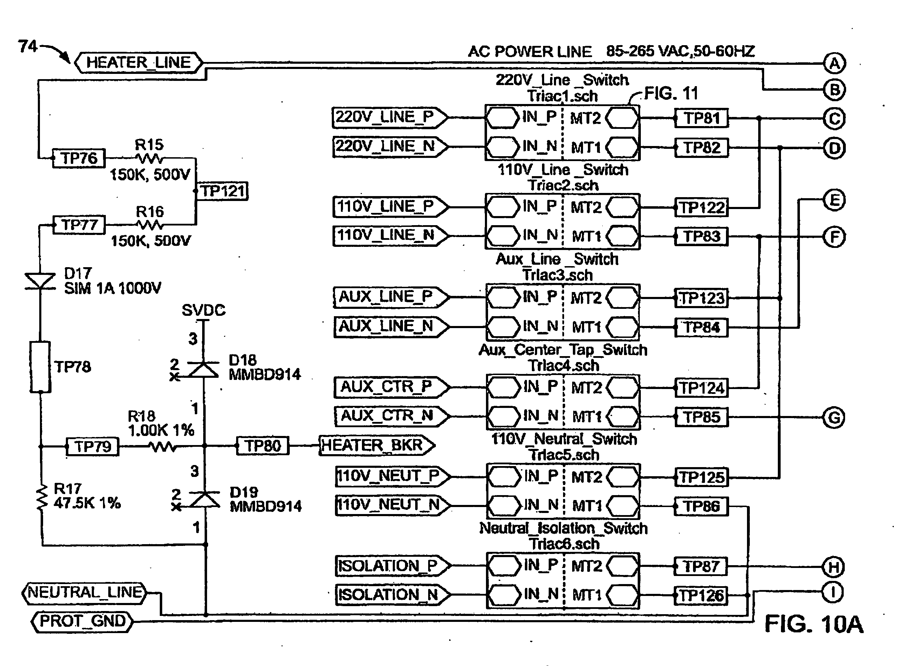

In the heater control logic of Fig. 9, a heater control and status isolated interface 80 is provided by the four opto-couplers shown in Fig. 9A. The HEATER_BKR input to the operational amplifier U4D comes via the rectified output of the circuit breaker in the heater tray from Fig. 10A of the relay control circuit. The portion of the heater control logic shown in Fig. 9B is designed to reset the flip-flop U3A on power up and inhibit output on low voltage. This circuit takes as inputs the DET_120_N output of the voltage detector and LINE_CLK of Fig. 8. Flip-flop U3A stores the state of the voltage until reset. The output of flip-flop U3A is applied via flip-flop U6 to a set of three gates U7A, U7B and U7C that produce the basic inputs to the relay circuit 74, namely 240V_ON, 110V_ON and AUX_ON. The 240V_ON and 110V_ON signals are gated by HEATER_ON signals generated by the I/O board beater ON/OFF controller 54 (Fig. 4-6) and passed from the connector J3 via the opto-coupler 80 in Fig. 9A. The AUX_ON signal, if used, is generated by the I/O board heater controller as well and passed from connector J3 via the opto-coupler as called for by the cycler control system.

The relay signals 82 for the auxiliary heater relays and signals 84 for the 120v line relays and 86 for the 220v line relays are shown in Fig. 9C, along with their associated service LED's.

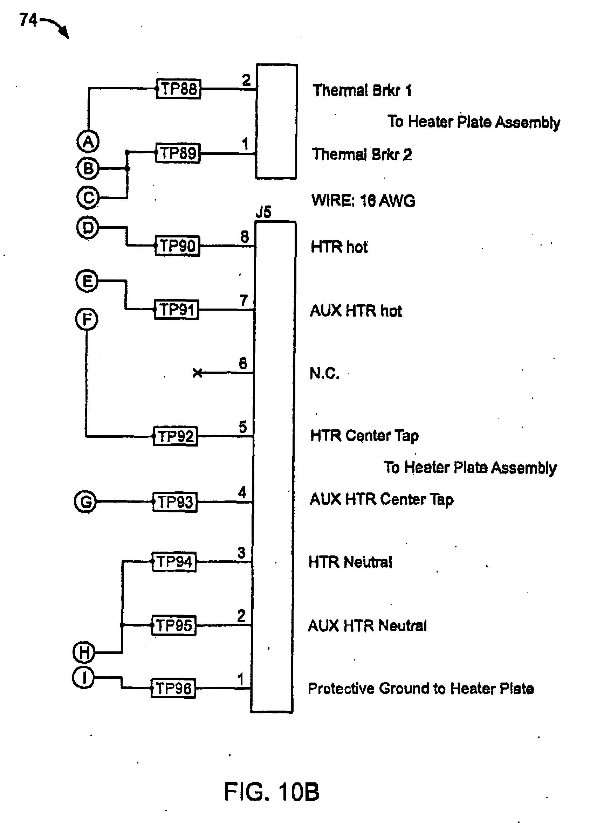

The relay control signals generated by the logic circuit of Fig. 9 are passed to the triac solid state switch array shown in Fig. 10A. Connections to the heater coils are indicated in Fig. 10B via the lines with the circled letters A-H. Note the heater plate assembly includes thermal circuit breaker with terminals 1 and 2. Thermal Bkr1 is connected directly to HEATER_LINE (i.e., line current from the power cord 30) and Thermal Bkr 2 in Fig. 10B is connected directly to the 220v and 110v line switches in Fig. 10A via line C. Thus current flows from the HEATER_LINE through the circuit breaker and back through either the 110 or 220 V Line switch before going to the center tap or end of the coil pair, depending on the state of the relays depending on line voltage, then through the coils and then returning to neutral through HTR Neutral in Fig. 10B.

The optional auxiliary heater coils, if activated by AUX_ON (Fig. 9B), operate the same way in parallel but have lower resistance than the main heater coils.

The invention has been described in terms of particular embodiments. Other embodiments are within the scope of the following claims. For example, while coil pairs are disclosed for the heater, any plurality of series connected coils which can be energized alternately in series or in parallel can be implemented. In addition, while the embodiments shown above involve a heater bag standing on a heater tray, a solution bag can empty its contents through an on-the-fly heater en route to the patient line, for example taking several maze like turns around a heating plate. The same solution for dual voltage adjustment can accommodate this flow through heating system as well as the stationary heater bag. Further, the above described embodiments are designed to be used with a PD cycler. However, the invention can be used on any type of peritoneal dialysis machine that preheats PD solution before infusion, The terms 110 VAC and 220 VAC used herein are intended to designate voltages within the ranges commonly encountered today as line current in the United States and Europe, respectively.

A number of embodiments of the invention have been described. Nevertheless, it will be understood that various modifications may be made without departing from the spirit and scope of the invention. Accordingly, other embodiments are within the scope of the following claims.

## Claims

1. A portable peritoneal dialysis machine, comprising a source of PD solution (18),
a patient line (16) for passing PD solution to and from the patient's abdominal cavity,
a controller (10) for delivering a predetermined quantity of PD solution to the patient's abdomen via the patient line,
a heater (22,56) for heating the PD solution before delivering it to the patient, wherein
the heater includes at least two heating elements (60,62) electrically connected in series via a center tap (64), **characterized in that**
the machine further comprises
a voltage detection circuit (44,76) to be connected to an incoming power line (30,70) for sensing whether the line voltage is 220 VAC or 110 VAC and producing an output indicative of line voltage, and
a switch circuit responsive to the voltage detection circuit output for applying 220 VAC across the heating elements (60,62) in series or 110 VAC via the center tap (64) through the elements in parallel, whereby approximately the same wattage is automatically produced by the heater under either 220 VAC or 110 VAC so that the PD solution is heated at approximately the same rate under either voltage.

2. The machine of claim 1, wherein the voltage detection circuit provides two complementary mutually exclusive logic outputs to the switch circuit, one indicating the presence of 220 VAC line current and the other indicating the presence of 110 VAC line current.

3. The machine of claim 1 or 2 wherein the heating elements are electrically interconnected resistive heating coils (60,62).

4. The machine of claim 1, 2 or 3 where in the source of PD solution is a PD solution bag (18), and further including a heater bag (20) and a tray (22,56) on the controller (10) for receiving said heater bag, the heater being operatively juxtaposed with the tray to warm the heater bag.

5. The machine of claim 1, 2, 3 or 4 wherein the heating elements are resistive heating coils (60,62) embedded in the tray (56).

6. The machine of any proceeding claim, wherein the heater includes a pair of auxiliary heating elements also connected in series via a center tap, the switch circuit further applying 220 VAC across both auxiliary heating elements in series or 110 VAC via the center tap through the elements in parallel, whereby approximately the same wattage is automatically produced by the auxiliary heating elements under either 220 VAC or 110 VAC.

7. The machine of any preceding claim, wherein the switch circuit includes a switch control circuit (52) and a plurality of switches (#1,#2,#3,#4) interconnecting the line voltage and neutral to the heating elements.

8. The machine of any preceding claim, wherein the switch control circuit is responsive to a separately generated heater ON/OFF signal to cause the switches to connect the line voltage and neutral to the heating elements to start or stop warming the PD solution.

9. The machine of claim 7 or 8, wherein the plurality of switches includes first, second, third and fourth switches, the first switch (#1) connecting 220 line voltage when closed to the end of one of the heating elements (60), the second switch (#2) connecting the end of one of the heating elements (60) to neutral when closed, the third switch (#3) connecting 110 line voltage to the center tap (64) when closed and the fourth switch (#4) connecting the end of the other heating element (62) to neutral when closed, the switch control circuit (52) closing the second, third and fourth switches when the output of the voltage detection circuit indicates that line voltage is 110 VAC, and closing the first and fourth switches when the output of the voltage detection circuit indicates that line voltage is 220 VAC.

10. The machine of claim 9, wherein the switch control circuit (52) is responsive to a separately generated heater ON/OFF signal to cause the switches to connect the line voltage and neutral to the heating elements to start or stop warming the PD solution.

11. A method of preparing PD solution for performing peritoneal dialysis, comprising
providing a source ofPD solution (18),
providing a plurality of interconnected heating elements (60,62) arranged to heat the PD solution prior to infusion,
automatically detecting the line voltage,
automatically reconfiguring the connection between a plurality of interconnected heating elements (60,62) with the line voltage and neutral in response to the detected line voltage to supply the same wattage for heating the PD solution under at least two substantially different line voltages, and
heating the PD solution with the reconfigured heating elements.

12. The method of claim 11, wherein the heating elements (60,62) provided are connected in series via at least one junction (64), and the reconfiguring step automatically applies line voltage across the heating elements in series at one line voltage and in parallel at another line voltage.

## Patentansprüche

1. Tragbare Peritonealdialysemaschine, die Folgendes umfasst:
eine Quelle (18) von PD-Lösung,
eine Patientenleitung (16) zum Führen von PD-Lösung zu und von der Bauchhöhle des Patienten,
ein Steuergerät (10) zum Zuführen einer vorbestimmten Menge an PD-Lösung zum Bauch des Patienten über die Patientenleitung,
ein Heizgerät (22, 56) zum Erhitzen der PD-Lösung vor dem Zuführen derselben zu dem Patienten, wobei
das Heizgerät wenigstens zwei Heizelemente (60, 62) einschließt, die über eine Mittelanzapfung (64) in Reihe miteinander verbunden sind, **dadurch gekennzeichnet, dass** die Maschine ferner Folgendes umfasst:
einen Spannungserkermungsstromkreis (44, 76), der mit einer ankommenden Netzleitung (30, 70) zu verbinder ist, um abzufühlen, ob die Netzspannung 220 V AC oder 110 V AC beträgt, und eine Ausgabe zu erzeugen, welche die Netzspannung anzeigt, und
einen Schalterstromkreis, der auf die Ausgabe des Sparmungserkennungsstromkreises anspricht, um 220 V AC über die Heizelemente (60, 62) in Reihe oder 110 V AC über die Mittelanzapfung (64) durch die Elemente in Parallelschaltung anzulegen, wodurch automatisch sowohl unter 220 V AC als auch unter 110 V AC annähernd die gleiche Wattleistung durch das Heizgerät erzeugt wird, so dass die PD-Lösung unter beiden Spannungen mit annähernd der gleichen Geschwindigkeit erwärmt wird.

2. Maschine nach Anspruch 1, wobei der Spannungserkennungsstromkreis zwei komplementäre, einander wechselseitig ausschließende Logikausgaben an den Schalterstromkreis bereitstellt, wobei die eine das Vorhandensein von 220-V-AC-Netzstrom anzeigt und die andere das Vorhandensein von 110-V-AC-Netzstrom anzeigt.

3. Maschine nach Anspruch 1 oder 2, wobei die Heizelemente elektrisch miteinander verbundene Widerstandsheizspulen (60, 62) sind.

4. Maschine nach Anspruch 1, 2 oder 3, wobei die Quelle von PD-Lösung ein PD-Lösungsbeutel (18) ist und ferner einen Heizgerätbeutel (20) und eine Ablage (22, 56) auf dem Steuergerät (10) zum Aufnehmen des Heizgerätbeutels einschließt, wobei der Heizgerätbeutel wirksam mit der Ablage nebeneinandergesetzt ist, um den Heizgerätbeutel zu erwärmen

5. Maschine nach Anspruch 1, 2, 3 oder 4, wobei die Heizelemente in die Ablage (56) eingebettete Widerstandsheizspulen (60, 62) sind.

6. Maschine nach einem der vorhergehenden Ansprüche, wobei das Heizgerät ein Paar von Hilfsheizelementen einschließt, die ebenfalls über eine Mittelanzapfung in Reihe verbunden sind, wobei der Schalterstromkreis ferner 220 V AC über die beiden Hilfsheizelemente in Reihe oder 110 V AC über die Mittelanzapfung durch die Elemente in Parallelschaltung anlegt, wodurch automatisch sowohl unter 220 V AC als auch unter 110 V AC annähernd die gleiche Wattleistung durch die Hilfsheizelemente erzeugt wird.

7. Maschine nach einem der vorhergehenden Ansprüche, wobei der Schalterstromkreis einen Schalter-Steuerungsstromkreis (52) und mehrere Schalter (#1, #2, #3, #4) einschließt, welche die Netzspannung und den Neutralleiter mit den Heizelementen verbinden,

8. Maschine nach einem der vorhergehenden Ansprüche, wobei der Schalter-Steuerungsstromkreis auf ein gesondert erzeugtes Heizgerät-EIN/AUS-Signal anspricht, um zu bewirken, dass die Schalter die Netzspannung und den Neutralleiter mit den Heizelementen verbinden, um das Erwärmen der PD-Lösung zu beginnen oder zu beenden.

9. Maschine nach Anspruch 7 oder 8, wobei die mehreren Schalter einen ersten, einen zweiten, einen dritten und einen vierten Schalter einschließen, wobei der erste Schalter (#1), wenn er geschlossen ist, die 220-V-Netzspannung mit dem Ende des einen der Heizelemente (60) verbindet, der zweite Schalter (#2), wenn er geschlossen ist, das Ende des einen der Heizelemente (60) mit dem Neutralleiter verbindet, der dritte Schalter (#3), wenn er geschlossen ist, die 110-V-Netzspannung mit der Mittelanzapfung (64) verbindet und der vierte Schalter (#4), wenn er geschlossen ist, das Ende des anderen Heizelementes (62) mit dem Neutralleiter verbindet, wobei der Schalter-Steuerungsstromkreis (52) den zweiten, den dritten und den vierten Schalter schließt, wenn die Ausgabe des Spannungserkennungsschaltkreises anzeigt, dass die Netzspannung 110 V AC beträgt, und den ersten und den vierten Schalter schließt, wenn die Ausgabe des Spannungserkennungsschaltkreises anzeigt, dass die Netzspannung 220 V AC beträgt.

10. Maschine nach Anspruch 9, wobei der Schalter-Steuerungsstromkreis (52) auf ein gesondert erzeugtes Heizgerät-EIN/AUS-Signal anspricht, um zu bewirken, dass die Schalter die Netzspannung und den Neutralleiter mit den Heizelementen verbinden, um das Erwärmen der P17-Lösung zu beginnen oder zu beenden.

11. Verfahren zum Bereiten von PD-Lösung zum Durchführen einer Peritonealdialyse, das Folgendes umfasst:
das Bereitstellen einer Quelle (18) von PD-Lösung,
das Bereitstellen mehrerer miteinander verbundener Heizelemente (60, 62), die zum Erhitzen der PD-Lösung vor der Infusion angeordnet sind,
das automatische Erkennen der Netzspannung,
das automatische Neukonfigurieren der Verbindung zwischen mehreren miteinander verbundenen Heizelementen (60, 62) mit der Netzspannung und dem Neutralleiter als Reaktion auf die erkannte Netzspannung, um unter wenigstens zwei im Wesentlichen unterschiedlichen Netzspannungen die gleiche Wattleistung zum Erhitzen der PD-Lösung bereitzustellen, und
das Erhitzen der PD-Lösung mit den neu konfigurierten Heizelementen.

12. Verfahren nach Anspruch 11, wobei die bereitgestellten Heizelemente (60, 62) über wenigstens eine Verbindung (64) in Reihe miteinander verbunden sind und der Neukonfigurationsschritt automatisch die Netzspannung über die Heizelemente bei einer Netzspannung in Reihe und bei einer anderen Netzspannung in Parallelschaltung anlegt.

## Revendications

1. Machine de dialyse péritonéale portative, comprenant :
une source de solution PD (dialyse péritonéale) (18) ;
une ligne de raccordement du patient (16) pour faire passer la solution PD vers la cavité abdominale du patient et à partir de celle-ci ;
un moyen de commande (10) pour administrer une quantité prédéterminée de solution PD à l'abdomen du patient à travers la ligne de raccordement du patient ;
un dispositif de chauffage (22, 56) pour chauffer la solution PD avant son administration au patient, dans laquelle
le dispositif de chauffage englobe au moins deux éléments de chauffage (60, 62) connectés électriquement en série par l'intermédiaire d'une prise centrale (64) ; et **caractérisée en ce que** la machine comprend en outre :
un circuit de détection de la tension (44, 76) destiné à être connecté à une ligne électrique rentrante (30, 70), pour détecter si la tension de ligne correspond à 220 VAC ou à 110 VAC et transmettant un signal de sortie indicatif de la tension de ligne ; et
un circuit de commutation réagissant au signal de sortie du circuit de détection de la tension pour appliquer une tension de 220 VAC à travers les éléments de chauffage (60, 62) connectés en série, ou une tension de 110 VAC par l'intermédiaire de la prise centrale (64) à travers les éléments connectés en parallèle, une puissance pratiquement égale étant ainsi produite automatiquement par le dispositif de chauffage en présence d'une tension de 220 VAC ou de 110 VAC, de sorte que la solution PD est chauffée pratiquement à la même vitesse en présence de chaque tension.

2. Machine selon la revendication 1, dans laquelle le circuit de détection de la tension transmet deux signaux de sortie logiques mutuellement exclusifs complémentaires au circuit de commutation, l'un indiquant la présence d'un courant de ligne de 220 VAC et l'autre indiquant la présence d'un courant de ligne de 110 VAC.

3. Machine selon les revendications 1 ou 2, dans laquelle les éléments de chauffage sont des serpentins de chauffage à résistance interconnectés (60, 62).

4. Machine selon les revendications 1, 2 ou 3, dans laquelle la source de solution PD est une poche de solution PD (18), et englobant en outre une poche de chauffage (20) et un plateau (22, 56) sur le moyen de commande (10) pour recevoir ladite poche de chauffage, le dispositif de chauffage étant juxtaposé opérationnellement au plateau pour chauffer la poche de chauffage.

5. Machine selon les revendications 1, 2, 3 ou 4, dans laquelle les éléments de chauffage sont des serpentins de chauffage à résistance (60, 62) noyés dans le plateau (56).

6. Machine selon l'une quelconque des revendications précédentes, dans laquelle le dispositif de chauffage englobe une paire d'éléments de chauffage auxiliaires, connectés également en série par l'intermédiaire d'une prise centrale, le circuit de commutation appliquant en outre une tension de 220 VAC à travers les deux éléments de chauffage auxiliaires connectés en série, ou une tension de 110 VAC par l'intermédiaire de la prise centrale à travers les éléments connectés en parallèle, pratiquement la même puissance étant ainsi produite automatiquement par les éléments de chauffage auxiliaires en présence d'une tension de 220 VAC ou de 110 VAC.

7. Machine selon l'une quelconque des revendications précédentes, dans laquelle le circuit de commutation comprend un circuit de commande de commutation (52) et plusieurs commutateurs (# 1, #2, #3, #4) assurant l'interconnexion de la tension de ligne et du neutre aux éléments de chauffage.

8. Machine selon l'une quelconque des revendications précédentes, dans laquelle le circuit de commande de commutation réagit à un signal EN/HORS du dispositif de chauffage, transmis séparément, pour entraîner les commutateurs à connecter la tension de ligne et le neutre aux éléments de chauffage en vue de démarrer ou d'arrêter l'échauffement de la solution PD.

9. Machine selon les revendications 7 ou 8, dans laquelle les plusieurs commutateurs englobent des premier, deuxième, troisième et quatrième commutateurs, le premier commutateur (#1) connectent la tension de ligne de 220 lors de sa fermeture à l'extrémité de l'un des éléments de chauffage (60), le deuxième commutateur (#2) connectant l'extrémité de l'un des éléments de chauffage (60) au neutre lors de sa fermeture, le troisième commutateur (#3) connectant la tension de ligne de 110 à la prise centrale (64) lors de sa fermeture, et le quatrième commutateur (#4) connectant l'extrémité de l'autre élément de chauffage (62) au neutre lors de sa fermeture, le circuit de commande de commutation (52) fermant les deuxième, troisième et quatrième commutateurs lorsque le signal de sortie du circuit de détection de la tension indique que la tension de ligne correspond à 110 VAC, et fermant les premier et quatrième commutateurs lorsque le signal de sortie du circuit de détection de la tension indique que la tension de ligne correspond à 220 VAC.

10. Machine selon la revendication 9, dans laquelle le circuit de commande de commutation (52) réagit à un signal EN/HORS du dispositif de chauffage, transmis séparément, pour entraîner les commutateurs à connecter la tension de ligne et le neutre aux éléments de chauffage, pour démarrer ou arrêter l'échauffement de la solution PD.

11. Procédé de préparation d'une solution PD pour exécuter une dialyse péritonéale, comprenant les étapes ci-dessous :
fourniture d'une source de solution PD (18) ;
fourniture de plusieurs éléments de chauffage interconnectés (60, 62), destinés à chauffer la solution PD avant la perfusion ;
détection anatomique de la tension de ligne ;
reconfiguration automatique de la connexion entre plusieurs éléments de chauffage interconnectés (60, 62) à la tension de ligne et au neutre en réponse à la tension de ligne détectée, pour fournir la même puissance pour chauffer la solution PD en présence d'au moins deux tensions de ligne notablement différentes ; et
chauffage de la solution PD par l'intermédiaire des éléments de chauffage reconfigurés.

12. Procédé selon la revendication 11, dans lequel les éléments de chauffage (60, 62) fournis sont connectés en série par l'intermédiaire d'au moins une jonction (64), l'étape de reconfiguration appliquant automatiquement la tension de ligne à travers les éléments de chauffage connectés en série en présence d'une tension de ligne et en présence d'une autre tension de ligne pour les éléments de chauffage connectés en parallèle.
